# EUROPEAN PATENT SPECIFICATION

(11) **EP 2 167 150 B1**
(45) Date of publication and mention of the grant of the patent: **22.12.2010**
(21) Application number: 08776302.5
(22) Date of filing: 27.06.2008
(51) Int. Cl.: A61L 27/46, A61L 27/42, A61L 27/56

(54) **POROUS COMPOSITE MATERIAL, PREPARATION PROCESS THEREOF AND USE TO REALIZE TISSUE ENGINEERING DEVICES**
PORÖSER VERBUNDWERKSTOFF, HERSTELLUNGSVERFAHREN DAFÜR UND SEINE VERWENDUNG ZUR HERSTELLUNG VON GEWEBEZÜCHTUNGSVORRICHTUNGEN
MATÉRIAU COMPOSITE POREUX, PROCÉDÉ DE PRÉPARATION ET UTILISATIONS EN VUE DE L'OBTENTION DE DISPOSITIFS GÉNÉRATEURS DE TISSUS

(30) Priority: 29.06.2007 IT MI20071298
(43) Date of publication of application: 31.03.2010
(73) Proprietor: Alma Mater Studiorum -Universita' di Bologna, 40126 Bologna (IT); Istituto Ortopedico Rizzoli, 40136 Bologna (IT)
(72) Inventor: BIGI, Adriana, I-40100 Bologna (IT); PANZAVOLTA, Silvia, I-47100 Forli (IT); GIARDINO, Roberto, I-40137 Bologna (IT); FINI, Milena, I-40068 San Lazzaro Savena-Bologna (IT)
(74) Representative: Bottero, Carlo
(86) International application number: PCT/IB2008/001688
(87) International publication number: WO 2009/004445

(56) References cited:
- WO-A-03/071991
- WO-A-2005/081699
- WO-A-2006/031196
- DATABASE CA [Online] CHEMICAL ABSTRACTS SERVICE, COLUMBUS, OHIO, US; NAKANO, YUMIKO ET AL: "In vitro and in vivo characterization and mechanical properties of .alpha.-TCP/OCP settings" XP002553524 retrieved from STN Database accession no. 2000:47418 & BIOCERAMICS, PROCEEDINGS OF THE INTERNATIONAL SYMPOSIUM ON CERAMICS IN MEDICINE , 12, 315-318 CODEN: BPCMFX, 1999,
- A. BIGI ET AL.: "ALPHA-TRICALCIUM PHOSPHATE HYDROLYSIS TO OCTACALCIUM PHOSPHATE: EFFECT OF SODIUM POLYACRYLATE" BIOMATERIALS, vol. 23, 2002, pages 1849-1854, XP002553521 cited in the application
- Y. TAKAHASHI ET AL.: "Osteogenic differrentiation of mesenchymal stem cells in biodegradable sponges composed of gelatin and beta-tricalcium phosphate" BIOMATERIALS, vol. 26, 2005, pages 3587-3596, XP002553522 cited in the application
- CHUN-HSU YAO: "Calvarial bone response to a tricalcium phosphate-genipin crosslinked gelatin composite" BIOMATERIALS, vol. 26, 2005, pages 3065-3074, XP002553523 cited in the application

## Description

The present invention refers to a porous composite material, the preparation process thereof and its use for osseous bone and/or bone-cartilage regeneration and to realize tissue engineering devices.

As it is understood, bone tissue is an extremely complex biomineralized composite material, mainly consisting of inorganic components such as hydroxyapatite (HA) and water (70-80%) and of organic components such as type I collagen, proteoglycans and other non-collagen proteins (20-30%). During bone formation, low cristallinity hydroxyapatite nanocrystals accumulate and intimately associate on the organic component in its fibrous form, such that they form a nanostructured composite material of excellent mechanic and elastic properties.

The bone defect and relative need of missing volume reintegration or need of existing volume increment constitutes a major challenge in the orthopaedic, maxillo-facial and neurosurgical field. Various biomaterials have been investigated and proposed as bone substitutes, which have to show high biocompatibility properties and concurrently such biomimetic characteristics as to activate biological mechanisms with host bone tissues and their cellular components, promoting the new-formation and bone consolidation processes. When this function has been completed, these materials are usually completely reabsorbed, leaving exclusive space to new-formed bone. This regeneration process is usually indicated as "guided bone regeneration".

For example, in the International Patent Application WO 03/071991 a porous matrix is described, which can be used as bone regeneration material, consisting of a fibrillar polymer, insoluble in water, especially an insoluble collagen, a collagen derivate or a modified gelatin derivate, mineralized with calcium phosphate. The biopolymer may be used mixed with a water soluble ligand, for example soluble collagen, gelatin, polylactic acid, polyglycolic acid and others. The mineralization has been obtained by treating the polymer fibres with a calcium ions and phosphate ions aqueous solution with basic pH. Then the water soluble ligand has been added to and mixed with the mineralized biopolymer aqueous solution; then the resulting mixture has been cooled and freeze-dried. The porous matrix may be crosslinked by adding, for example, glutaraldehyde.

International Patent Application WO 06/031196 discloses a porous composite consisting of a biomaterial and a mineral charge. The biomaterial may be selected from a wide range of products, comprising proteins (for example, collagen, elastin, gelatin and others), peptides, polysaccharides. The mineral charge may be calcium phosphate, for example apatite or substituted apatite, or brushite, tricalcium phosphate, octacalcium phosphate. The biomineral may be crosslinked with various crosslinking agents, such as acrylamides, dions, glutaraldehyde, acetaldehyde, formaldehyde or ribose. The composite may be prepared by mixing the biomaterial and mineral charge in water according to various methods, to obtain a suspension, which is then freeze-dried.

Chun-Hsu Yao et al. "Calvarial bone response to tricalcium phosphate-genipin crosslinked gelatin composite", Biomaterials 26 (2005), p. 3065-3074, reports a study on the biological response in vivo of a porous biodegradable composite obtained from crosslinked gelatin with genipin and tricalcium phosphate ceramic particles. A 0,5 w/% genipin aqueous solution has been added to a 18% gelatin aqueous solution to produce gelatin crosslinking. Subsequently, tricalcium phosphate has been added in the form of particles, with a size of 200-300 µm (from Merck). After solidification, the composite has been freezed at -80°C and freeze-dried.

Yoshitake Takahashi et al. "Osteogenic differentiation of mesenchymal stem cells in biodegradable sponges composed of gelatin and β-tricalcium phosphate", Biomaterials 26 (2005), p. 3587-3596, describes the preparation of biodegradable porous materials consisting of gelatin and β-tricalcium phosphate and their use for in vitro osteogenic differentiation of mesenchymal stem cells. These materials have been prepared by crosslinking of gelatin with glutaraldehyde in the presence of β-tricalcium phosphate and subsequent freeze-drying.

Hae-Won Kim et al. "Stimulation of osteoblast responses to biomimetic nanocomposites of gelatin-hydroxyapatite for tissue engineering scaffolds", Biomaterials 26 (2005), p. 5221-5230, finally regards a study on the in vitro response of osteoblastic cells in presence of a collagen/hydroxyapatite based nanocomposite. The nanocomposite has been prepared by co-precipitation of hydroxyapatite with a gelatin solution and subsequent freeze-drying. The hydroxyapatite may be obtained by adding calcium ions and phosphate ions to the gelatin solution, or by mixing directly hydroxyapatite as a powder with the gelatin solution.

The Applicants have aimed to obtain a porous composite material usable to accelerate bone and/or bone-cartilage regeneration, showing a suitable in vivo resorption rate, proportioned to the processes of rapid new tissue formation, so that said composite material is especially suited to carry out bone and/or bone-cartilage regeneration techniques and to realize tissue engineering devices.

The Applicants have surprisingly found that this problem may be solved by a porous composite material as claimed by the following claims, wherein at least one interdispersed biopolymer is present having a calcium-phosphatic mineral component comprising from 50 w/% to 95 w/% of α-tricalcium phosphate (α-TCP, α-Ca₃(PO₄)₂) and from 5 w/% to 50 w/% of octacalcium phosphate (OCP, Ca₈H₂(PO₄)₆·5H₂O) to the total weight of the mineral component. Said combination of α-TCP and OCP allows for an increased in vivo resorption rate and thereby for a faster formation of new bone tissue having a low cristallinity mineral component with nanocrystalline structure, said features being very similar to the features of biologic apatites. Therefore, according to a first aspect, the present invention refers to a porous composite material comprising at least one interdispersed biopolymer with a mineral component comprising from 50 w/% to 95 w/% of α-tricalcium phosphate (α-TCP) and from 5 w/% to 50 w/% of octacalcium phosphate (OCP), to the total weight of the mineral component.

Preferably, the mineral component comprises from 60 w/% to 85 w/% of α-TCP and from 15/% to 40 w/% of OCP. More preferably, the mineral component comprises from 70 w/% to 80 w/% of α-TCP and from 20 w/% to 30 w/% of OCP.

Preferably, the biopolymer is a protein or a polysaccharide. More preferably, the biopolymer is a water soluble protein, in particular animal gelatin obtained, for example, by extraction from biological tissue such as muscle, connective tissue, for example bone, tendon, ligament or cartilage, or skin or derma. The porous composite material preferably comprises from 30 w/% to 99 w/%, more preferably from 55 w/% to 95 w/%, of said at least one biopolymer, and from 1 w/% to 70 w/%, more preferably from 5 w/% to 45 w/% of the mineral component as defined above, the percentage being expressed with regard to the total weight of the porous composite material.

According to a further aspect, the present invention refers to a process for preparing a porous composite material as disclosed above comprising:
mixing the at least one biopolymer with a mineral component substantially consisting of α-tricalcium phosphate (α-TCP) in an aqueous medium so as to obtain a foam;
allowing the so obtained foam to stay for a sufficient time to obtain gelation of the biopolymer;
cooling the foam at a temperature lower than -20°C, preferably lower than -90°C;
freeze-drying the cooled foam.

According to a further aspect, the present invention refers to the use of a porous composite material as disclosed above as a material for bone and/or bone-cartilage regeneration.

According to a further aspect, the present invention refers to the use of a porous composite material as disclosed above as a material for the production of tissue engineering devices.

According to a further aspect, the present invention refers to the use of a porous composite material as disclosed above as a bone and/or bone-cartilage substitute (scaffold).

In accordance with the process according to the present invention, the α-TCP and OCP combination as defined above is obtained from α-TCP since α-TCP in an aqueous environment is partially hydrolyzed to OCP. To this regard, see A. Bigi et al. "α-Tricalcium phosphate hydrolysis to octacalcium phosphate: effect of sodium polyacrylate", Biomaterials 23 (2002), p. 1849-1854.

It is to be noted that such result is not obtainable from other calcium phosphates, for example from β-TCP, which is a crystallographic form of TCP completely different from α-TCP, neither from hydroxyapatite, which is the main constituent of commercial products generically marketed as TCP, as showed from the experimentation carried out by the Applicants and reported hereinafter.

According to a preferred embodiment, said at least one biopolymer present in the porous composite material according to the present invention is crosslinked. Thereby, it is possible to modulate the characteristic of high mechanic pressure resistance and greater degradation resistance following the application needs.

The crosslinking of the biopolymer may be obtained by adding at least one crosslinking agent during the preparation. The crosslinking agent may be selected, for example, from: amides, such as acrylamide; aldhehydes, such as glutaraldehyde; dions.

A particularly preferred crosslinking agent is genipin, a biodegradable natural product having very low cytotoxicity. Genipin is the product of hydrolysis of geniposide, usually obtained from the fruit of Gardenia jasminoides Ellis.

The crosslinking agent is added to the aqueous medium in which biopolymer and α-TCP are dispersed, while stirring for a sufficient time to obtain the crosslinking of the biopolymer. The crosslinking agent amount is usually from 0.5 w/% to 5 w/%, preferably from 1.5 w/% and 3.0 w/%, to the weight of the biopolymer.

Before cooling and freeze-drying, the obtained foam is allowed to stay for a sufficient time to achieve gelation of the biopolymer.

To modulate shape and size of the final material so that it is suitable for the desired use, the gelation phase may be carried out in a suitably shaped die. Thereby, wastes are minimized. Alternatively, to obtain the desired shape and size, it is possible to cut the material after freeze-drying.

The freeze-drying phase may be carried out by known techniques, at a temperature usually not over -20°C, preferably between -40 and -60°C, for a time usually not less than 18 hours, preferably from 24 hours to 3 days, under reduced pressure, usually lower than 10 millibar, preferably from 0,1 and 1,0 millibar.

The composite material according to the present invention shows a porous structure having a mean particle size from 1 to 500 µm. By SEM analysis, the porous structure shows both macro-porosity and micro-porosity, with interconnected macropores having a mean particles size from 100 to 200 µm. The macropores walls are microporous, the micropores mean particle size being a few µm.

The porous composite material according to the present invention may comprise cells for in situ e/o in vitro tissue engineering. These cells, differentiated (such as osteoblasts, osteocytes, chondroblasts, chondrocytes) and/or undifferentiated (such as mesenchymal stem cells) autologous or homologous, may be associated with the porous composite material during the surgical implant phase or they may be cultivated thereon to obtain in vitro engineered structures which will be implanted in vivo. Growth factors or other proteins and/or biological stimulators (both synthesized and biological, autologous or homologous), may be associated with the porous composite material during the production phase thereof, concurrently with the surgical implant with or without cells, and during the in vitro construct engineering phase before the implant.

The present invention will be presently illustrated by a few examples, which are not to be considered in any way limitating the scope of the invention.

The appended Figures illustrate:
Fig. 1 Various enlargements of SEM images of a gelatin porous material which does not contain the mineral component;
Fig. 2, 3, 4 e 5: various enlargements of SEM images of the porous composite material according to the present invention, which contains the mineral component in an amount of 9 w/% (Fig.2), 23 w/% (Fig. 3), 33 w/% (Fig. 4), 42 w/% (Fig.5) respectively, to the total amount of the porous composite material;
Fig. 6: X-ray diffraction diagram of α-TCP powders used for the preparation of the porous composite material of the present invention;
Fig. 7, 8, 9: X-ray diffraction diagram of powders of the mineral component, which is isolated from porous composite materials of the present invention containing the mineral component in an amount of 23 w/% (Fig. 7), 33 w/% (Fig. 8), 42 w/% (Fig. 9) respectively, to the total amount of the porous composite material;
Fig. 10: X-ray diffraction diagram of β-TCP powders used for preparing a porous composite material according to known art;
Fig. 11: X-ray diffraction diagram of powders of the mineral component isolated from porous composite material obtained from β-TCP according to the known art, said mineral component being present in an amount of 33 w/%, to the total weight of the porous composite material;
Fig. 12: X-ray diffraction diagram of powders of the commercial product TCP (Merck) used for preparing a porous composite material according to the known art;
Fig. 13: X-ray diffraction diagram of powders of the mineral component isolated from the porous composite material obtained from commercial product TCP (Merck) according to the known art, said mineral component being present in an amount of 42 w/%, to the total weight of the porous composite material.

### EXAMPLES 1-5

### Materials employed

Pig skin gelatin has been used, obtained by acid extraction.

α-TCP has been prepared by solid state reaction of a mixture of CaCCO₃ with CaHPO₄ ·2H₂O with a molar ratio of 1:2 at 1300°C for 5 hours. The solid product has been finely grinded before using.

### Porous composite material preparation

The various samples preparation was conducted according to the following phases.
a) The gelatin was dissolved in water containing α-TCP at concentrations such as to obtain a mineral component amount, in the final composite material, of 9 w/% (Example 2), of 23 w/% (Example 3), of 33 w/% (Example 4) and of 42 w/% (Example 5). Dissolution was obtained by mechanic stirring at 40°C for 50 minutes at 1,000 rpm. At the end of the stirring a foam was obtained.
b) The foam was gelled while keeping it in a Petri dish at ambient temperature for a time ranging from 10 to 40 minutes.
c) The obtained gel was frozen by immersion in liquid nitrogen (-195°C) for 10 minutes.
d) The frozen gel was freeze-dried at -50°C for 24 hours at a 1 millibar pressure.

A porous material was also prepared as a reference, using gelatin without adding the α-TCP, following the same methods as noted before. (Example 1)

If samples of crosslinked composite material are desired, after the (a) phase, a genipin aqueous solution may be added so as to obtain a genipin amount of 1,5 w/% to the gelatin weight. The so obtained composition is then kept under stirring for 10 minutes.

### Composite materials characterization

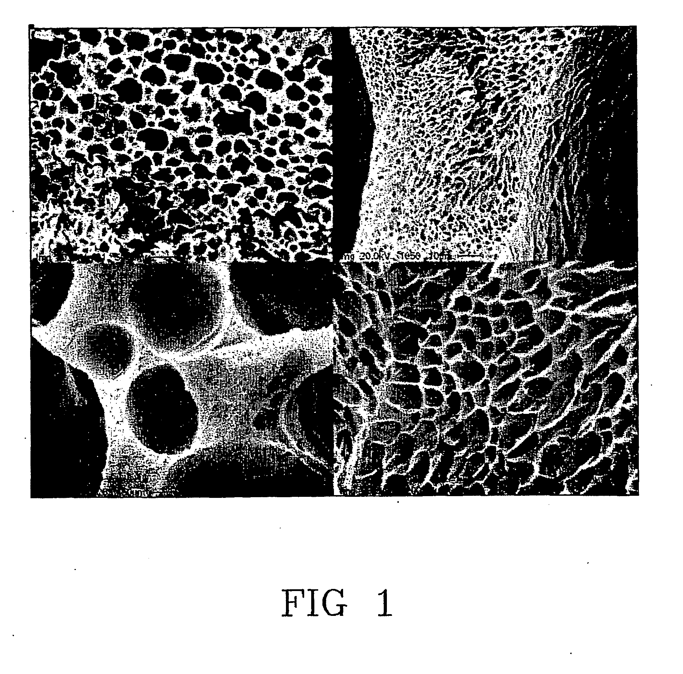

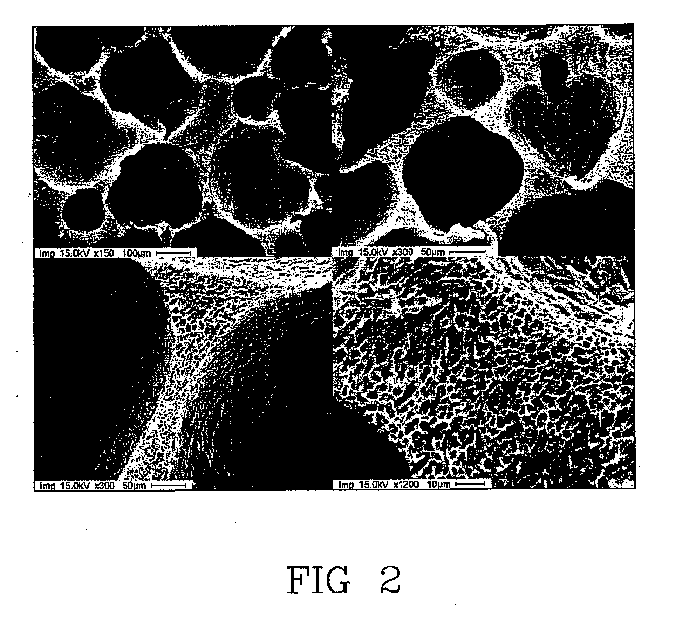

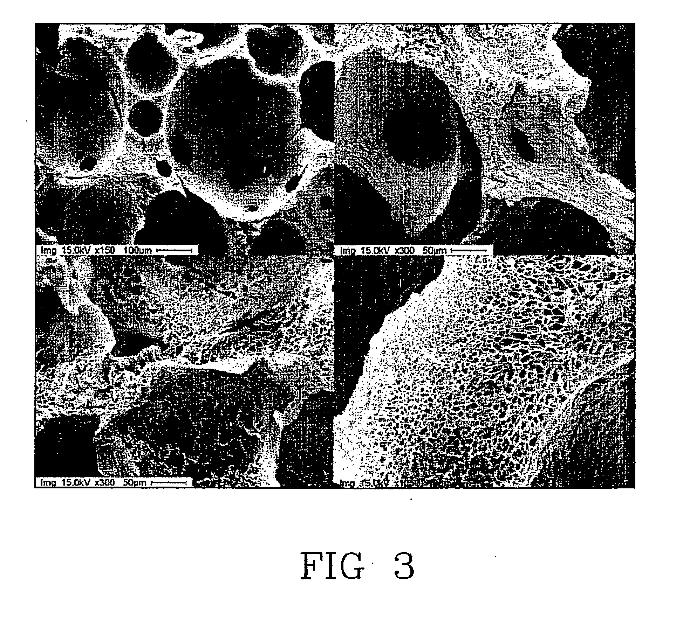

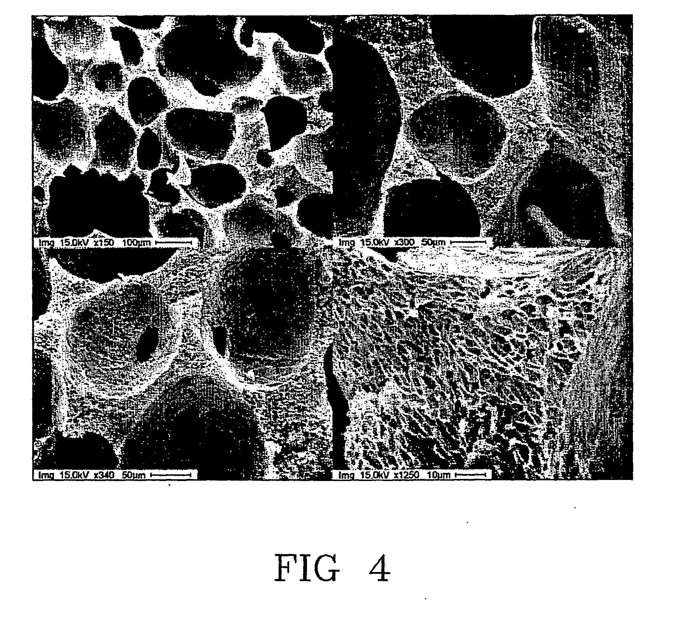

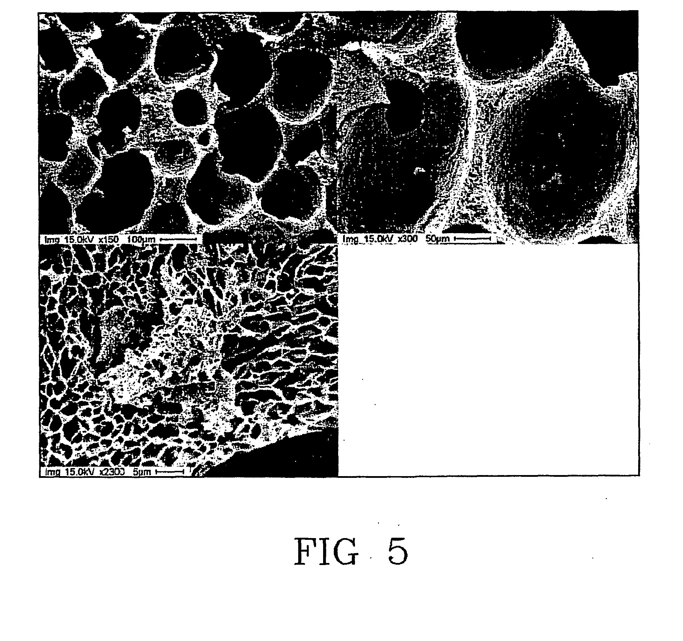

Figg. 2-5 images are SEM micrographs of porous composite material according to the present invention comprising the mineral component in amounts of: 9 w/% (Example 2, Fig. 2), 23 w/% (Example 3, Fig. 3); 33 w/% (Example 4, Fig. 4), 42 w/% (Example 5, Fig. 5). As a reference, Fig. 1 illustrates_SEM images of the gelatin porous material according to Example 1, not containing the mineral component, at various enlargements.

As can be noted, the porous structure exhibits a macro- and micro-porosity. The macropores, which seemed interconnected, had a mean particle size of 100-200 µm. The images do not show details pertaining to the inorganic phase, showing an excellent homogenization of the composite material components The characterization of the crystalline structure of the mineral component was carried out by X-ray diffraction analysis of powders, using a PANalytical X'Pert PRO diffractometer.

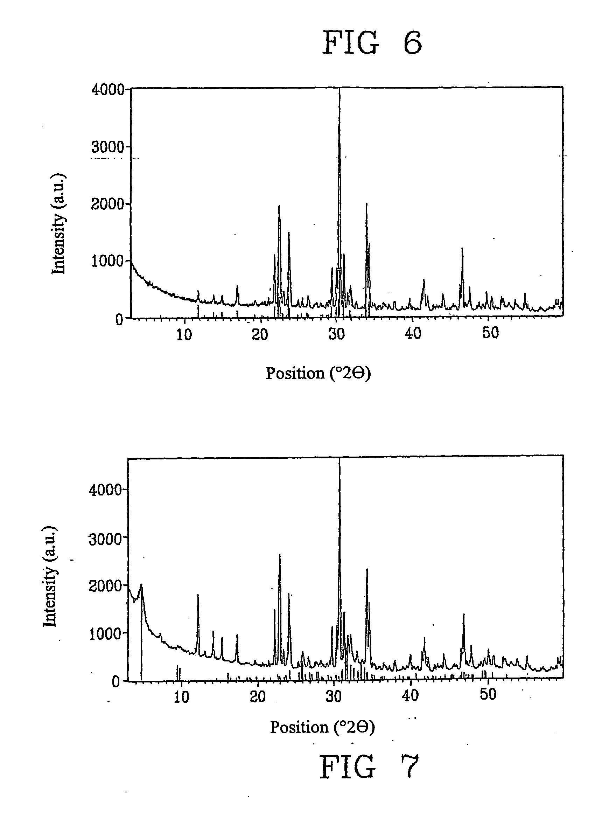

Fig. 6 shows the X-ray diffraction diagram of powders obtained from α-TCP used for preparing porous composite material samples. All the diffraction peaks coincide with those characteristic of α-TCP (in the diagram the α-TCP reference file ICDD is reported by segments corresponding to characteristic peaks).

Fig. 7 shows the X-ray diffraction diagram of powders obtained from the mineral component isolated from the composite material (by gelatin solubilization) containing 23 w/% of the mineral component immediately after freeze drying (Example 3). The diagram shows, as well as the typical α-TCP peaks, the presence of other diffraction peaks typical of OCP (in the diagram the ICDD reference file of OCP is reported by segments corresponding to characteristic peaks).

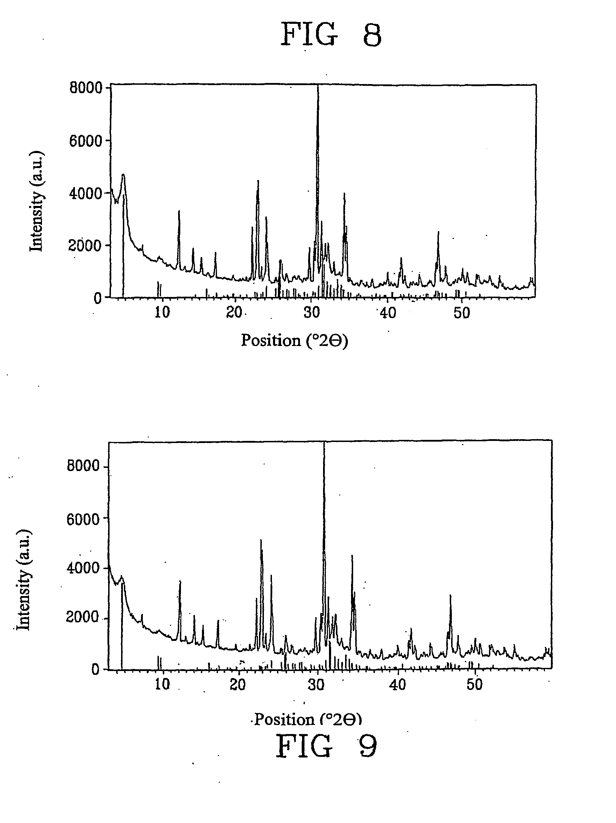

Similar results have been obtained for samples with different mineral component contents, as seen in Fig. 8 (33 w/%, Example 4) and Fig. 9 (42 w/%, Example 5). The relative amount of the two α-TCP and OCP phases, has been calculated by structural refinement of the whole diffraction diagram, realized by using the QUANTO program. Data obtained are very similar for all the examined samples; and mediated values of composite materials with different mineral component contents, examined at various time after preparation up to a month, are 26 ± 5% OCP and 74 ± 5% α-TCP.

The composite materials samples have been subjected to pressure by a INSTRON 4465 dynamometer equipped with a 1 KN load cell with a 1 mm/min bar speed. The results show how the mineral component content affects the mechanic properties under pressure. In fact, the mechanic properties increase as a function of the mineral component content: the stress value under pressure increases from the mean value of 0,08 ± 2 MPa, for samples free of mineral component, to the value of 0,21 ± 3 MPa for the samples with a 70 w/% mineral component content. Concurrently (simultaneously), the Young modulus value increases from 0,9±1 MPa to 4±1 MPa.

An intrusion porosimetric analysis was carried out on the above composite material samples by ThermoFinnigan Pascal 140 and Pascal 240 apparatus, using a maximum pressure of 240 MPa and a contact angle mercury-sample of 140°. Pore size has been also measured by SEM images. Results indicate interconnected porosity due to micro- and macropores, ranging in size from 1 to 500 µm.

### EXAMPLES 6-7 (according to known art)

Some preparing tests of a porous composite material have been carried out by the same methods as noted above for Examples 1-5, but using β-TCP as inorganic component, instead of α-TCP (Example 6) or the Merck commercial product indicated as TCP (Example 7).

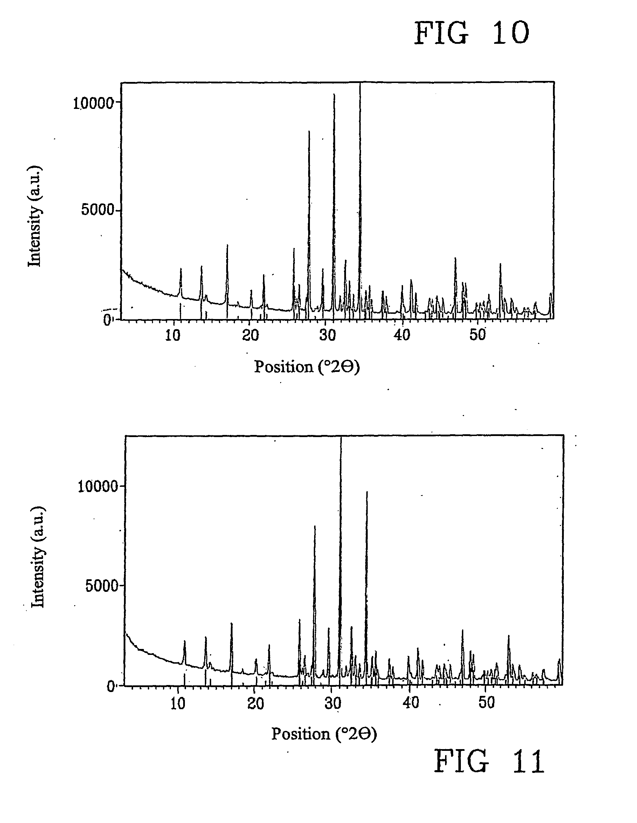

β-TCP has been prepared by solid state reaction of a mixture of CaCO₃ with CaHPO₄·2H₂O with a molar ratio 1:2 at 1000°C for 15 hours. The X-ray diffraction diagram of the objective product is illustrated in Fig. 10. All diffraction peaks coincide with those characteristic of β-TCP (in the diagram the ICDD reference file of β-TCP is reported by segments corresponding to characteristic peaks). Fig. 11 shows the X-ray diffraction diagram of powders obtained from the mineral component isolated from the composite material (by gelatin solubilizaton) containing 33 w/% of mineral component immediately after freeze-drying. All diffraction peaks coincide with those characteristic of β-TCP (the ICDD reference file of β-TCP is also reported by segments corresponding to characteristic peaks). A significant amount of OCP and α*-*TCP is not observed.

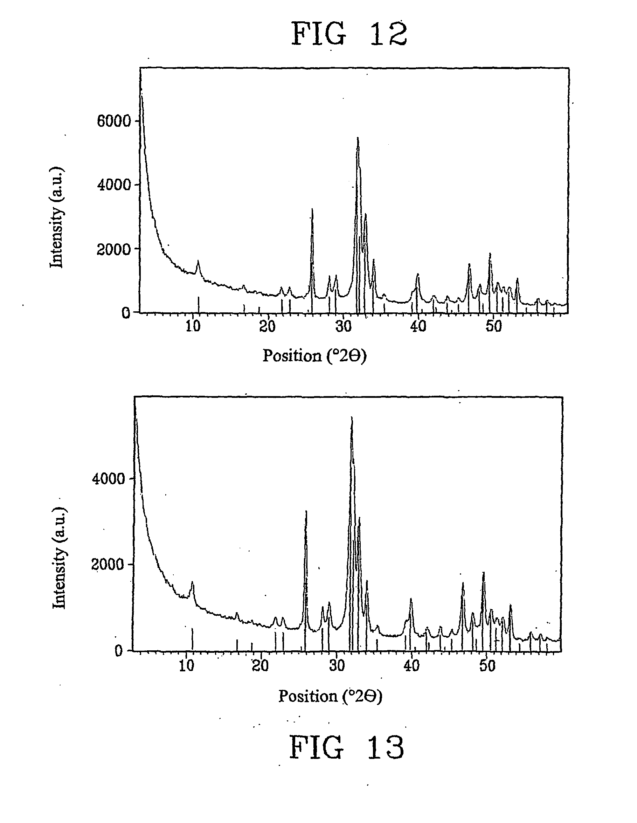

The X-ray diffraction diagram of the TCP commercial product (Merck) is reported in Fig. 12. All diffraction peaks coincide in fact with HA and not TCP characteristic peaks (the ICDD reference file of HA is reported in the diagram by segments corresponding to characteristic peaks). Fig. 13 shows the X-ray diffraction diagram of powders obtained from the mineral component isolated from the composite material (by gelatin solubilization) containing 42 w/% of the mineral component immediately after freeze-drying. All diffraction peaks coincide with HA characteristic peaks (in this case also the ICDD reference file of HA is reported in the diagram by segments corresponding to characteristic peaks). A significant amount of OCP and α-TCP is not observed.

## Claims

1. Porous composite material comprising at least one interdispersed biopolymer with a mineral component comprising from 50 w/% to 95 w/% of α-tricalcium phosphate (α-TCP) and from 5 w/% to 50 w/% of octacalcium phosphate (OCP), to the total weight of the mineral component.

2. Porous composite material according to claim 1, wherein the mineral component comprises from 60 w/% to 85 w/% of α-TCP and from 15 w/% to 40 w/% of OCP.

3. Porous composite material according to claim 2, wherein the mineral component comprises from 70 w/% to 80 w/% of α-TCP and from 20 w/% to 30 w/% of OCP.

4. Porous composite material according to any of the preceding claims, wherein the mineral component is obtained by partial hydrolysis in situ of α-TCP.

5. Porous composite material according to any of the preceding claims, wherein said at least one biopolymer is a protein or a polysaccharide.

6. Porous composite material according to any of the preceding claims, comprising from 30 w/% to 99 w/%, more preferably from 55 w/% to 95 w/%, of said at least one biopolymer, and from 1 w/% to 70 w/%, more preferably from 5 w/% to 45 w/%, of the mineral component.

7. Porous composite material according to any of the preceding claims, wherein said at least one biopolymer is crosslinked.

8. Porous composite material according to claim 7, wherein said at least one biopolymer is crosslinked by genipin.

9. Porous composite material according to any of the preceding claims, having a porous structure with mean particle size from 1 to 500 µm.

10. Porous composite material according to any of the preceding claims, further comprising differentiated and/or undifferentiated cells, autologous or homologous, growth factors or other proteins and/or biological stimulators.

11. Process for preparing a porous composite material according to any of the preceding claims, comprising:
mixing at least one biopolymer with a mineral component essentially composed of α-tricalcium phosphate (α-TCP) in an aqueous medium so as to obtain a foam;
leaving the so obtained foam for a sufficient time to obtain the biopolymer gelification;
cooling the foam at a temperature lower than -20°C,
preferably lower than -90°C;
freeze-drying the cooled foam.

12. Process according to claim 11, wherein at least one crosslinking agent is further added to the aqueous medium.

13. Process according to claim 12, wherein said crosslinking agent is genipin.

14. Process according to any of the claims from 11 to 13, wherein the freeze-drying phase is carried out at a temperature not over -20°C, preferably from -40 and -60°C, for a time not less than 18 hours, preferably between 24 hours and 3 days, under reduced pressure, less than 10 millibar, preferably from 0.1 and 1.0 millibar.

15. Use of a porous composite material according to any of the claims from 1 to 10 for the manufacture of a material for bone and/or bone-cartilage regeneration.

16. Use of a porous composite material according to any of the claims from 1 to 10 for the manufacture of a tissue engineering device.

17. Use of a porous composite material according to any of the claims from 1 to 10 for the manufacture of a bone and/or bone-cartilage substitute.

## Patentansprüche

1. Poröser Verbundwerkstoff, umfassend mindestens ein dazwischen dispergiertes Biopolymer mit einer Mineralkomponente, umfassend 50 Gew.-% bis 95 Gew.-% α-Tricalciumphosphat (α-TCP) und 5 Gew.-% bis 50 Gew.-% Octacalciumphosphat (OCP) zum Gesamtgewicht der Mineralkomponente.

2. Poröser Verbundwerkstoff nach Anspruch 1, wobei die Mineralkomponente 60 Gew.-% bis 85 Gew.-% α-TCP und 15 Gew.-% bis 40 Gew.-% OCP umfasst.

3. Poröser Verbundwerkstoff nach Anspruch 2, wobei die Mineralkomponente 70 Gew.-% bis 80 Gew.-% α-TCP und 20 Gew.-% bis 30 Gew.-% OCP umfasst.

4. Poröser Verbundwerkstoff nach einem der vorangehenden Ansprüche, wobei die Mineralkomponente in situ durch Partialhydrolyse eines α-TCP gewonnen wird.

5. Poröser Verbundwerkstoff nach einem der vorangehenden Ansprüche, wobei das mindestens eine Biopolymer ein Protein oder ein Polysaccharid ist.

6. Poröser Verbundwerkstoff nach einem der vorangehenden Ansprüche, umfassend 30 Gew.-% bis 99 Gew.-%, weiter bevorzugt 55 Gew.-% bis 95 Gew.-%, des mindestens einen Biopolymers, und 1 Gew.-% bis 70 Gew.-% , weiter bevorzugt 5 Gew.-% bis 45 Gew.-%, der Mineralkomponente.

7. Poröser Verbundwerkstoff nach einem der vorangehenden Ansprüche, wobei das mindestens eine Biopolymer quervernetzt ist.

8. Poröser Verbundwerkstoff nach Anspruch 7, wobei das mindestens eine Biopolymer durch Genipin quervernetzt ist.

9. Poröser Verbundwerkstoff nach einem der vorangehenden Ansprüche mit einer porösen Struktur mit einer mittleren Partikelgröße von 1 bis 500 µm.

10. Poröser Verbundwerkstoff nach einem der vorangehenden Ansprüche, ferner umfassend differenzierte und/oder undifferenzierte Zellen, autologe oder homologe, Wachstumsfaktoren oder andere Proteine und/oder biologische Stimulatoren.

11. Verfahren zur Herstellung eines porösen Verbundwerkstoffs nach einem der vorangehenden Ansprüche, umfassend:
Mischen mindestens eines Biopolymers mit einer Mineralkomponente, die sich im Wesentlichen aus einem
α-Tricalciumphosphat (α-TCP) in einem wässrigen Medium zusammensetzt, um einen Schaum zu erhalten;
Lassen des so erhaltenen Schaums für ausreichend Zeit,
um die Biopolymer-Gelbildung zu erhalten;
Abkühlen des Schaums bei einer Temperatur niedriger als -20 °C, vorzugsweise niedriger als -90 °C;
Gefriertrocknen des abgekühlten Schaums.

12. Verfahren nach Anspruch 11, wobei dem wässrigen Medium ferner mindestens ein Quervernetzungsmittel hinzugefügt wird.

13. Verfahren nach Anspruch 12, wobei das Quervernetzungsmittel Genipin ist.

14. Verfahren nach einem der Ansprüche 11 bis 13, wobei die Gefriertrocknungsphase bei einer Temperatur nicht über -20 °C, vorzugsweise zwischen -40 und -60 °C, in einem Zeitraum von nicht weniger als 18 Stunden, vorzugsweise zwischen 24 Stunden und 3 Tagen, unter reduziertem Druck, geringer als 10 Millibar, vorzugsweise zwischen 0,1 und 1,0 Millibar, ausgeführt wird.

15. Verwendung eines porösen Verbundwerkstoffs nach einem der Ansprüche 1 bis 10 für die Erzeugung eines Werkstoffs zur Knochen- und/oder Knochen-Knorpel-Regeneration.

16. Verwendung eines porösen Verbundwerkstoffs nach einem der Ansprüche 1 bis 10 für die Erzeugung einer Gewebezüchtungsvorrichtung.

17. Verwendung eines porösen Verbundwerkstoffs nach einem der Ansprüche 1 bis 10 für die Erzeugung eines Knochen- und/oder Knochen-Knorpel-Ersatzes.

## Revendications

1. Matériau composite poreux comprenant au moins un biopolymère interdispersé avec un composé minéral contenant de 50 p/% à 95 p/% de phosphate tricalcique α (α-TCP) et de 5 p/% à 50 p/% de phosphate octacalcique (OCP), à hauteur du poids total du composé minéral.

2. Matériau composite poreux selon la revendication 1, dans lequel le composé minéral contient de 60 p/% à 85 p/% de α-TCP et de 15 p/% à 40 p/% d'OCP.

3. Matériau composite poreux selon la revendication 2, dans lequel le composé minéral contient de 70 p/% à 80 p/% de α-TCP et de 20 p/% à 30 p/% d'OCP.

4. Matériau composite poreux selon l'une quelconque des revendications précédentes, dans lequel le composé minéral est obtenu à partir d'une hydrolyse partielle in situ de α-TCP.

5. Matériau composite poreux selon l'une quelconque des revendications précédentes, dans lequel ledit au moins un biopolymère est une protéine ou un polysaccharide.

6. Matériau composite poreux selon l'une quelconque des revendications précédentes, contenant de 30 p/% à 99 p/%, de préférence de 55 p/% à 95 p/%, dudit au moins un biopolymère, et de 1 p/% à 70 p/%, de préférence de 5 p/% à 45 p/%, du composé minéral.

7. Matériau composite poreux selon l'une quelconque des revendications précédentes, dans lequel ledit au moins un biopolymère est réticulé.

8. Matériau composite poreux selon la revendication 7, dans lequel ledit au moins un biopolymère est réticulé avec la génipine.

9. Matériau composite poreux selon l'une quelconque des revendications précédentes, avec une structure poreuse dont la valeur moyenne de la taille des particules va de 1 à 500 µm.

10. Matériau composite poreux selon l'une quelconque des revendications précédentes, comprenant également des cellules différenciées et/ou indifférenciées, autologues ou homologues, des facteurs de croissance ou des autres protéines et/ou des activateurs biologiques.

11. Procédé pour préparer un matériau composite poreux selon l'une quelconque des revendications précédentes, comprenant :
mélanger au moins un biopolymère avec un composé minéral constitué essentiellement de phosphate tricalcique α (α-TCP) dans un milieu aqueux pour obtenir une mousse ;
laisser reposer la mousse ainsi obtenue assez longtemps pour obtenir la gélification du biopolymère ;
refroidir la mousse à une température inférieure à - 20°C, de préférence inférieure à -90°C ;
lyophiliser la mousse refroidie.

12. Procédé selon la revendication 11, dans lequel au moins un agent réticulant est également ajouté au milieu aqueux.

13. Procédé selon la revendication 12, dans lequel ledit agent réticulant est la génipine.

14. Procédé selon l'une quelconque des revendications 11 à 13, dans lequel la phase de lyophilisation est réalisée à une température n'excédant pas -20°C, de préférence de -40 à -60°C, pendant une durée minimale de 18 heures, de préférence de 24 heures à 3 jours, sous pression réduite, moins de 10 millibars, de préférence de 0,1 à 1,0 millibar.

15. Utilisation d'un matériau composite poreux selon l'une quelconque des revendications 1 à 10 pour fabriquer un matériau régénérateur d'os et/ou de cartilage osseux.

16. Utilisation d'un matériau composite poreux selon l'une quelconque des revendications 1 à 10 pour fabriquer un dispositif générateur de tissus.

17. Utilisation d'un matériau composite poreux selon l'une quelconque des revendications 1 à 10 pour fabriquer un substitut osseux et/ou un substitut du cartilage osseux.
